Europäisches Patentamt

European Patent Office

Office européen des brevets

(19)

(11) Numéro de publication : **0 466 892 B1**

(12)                          **FASCICULE DE BREVET EUROPEEN**

(45) Date de publication du fascicule du brevet :
05.10.94 Bulletin 94/40

(51) Int. Cl.$^5$ : **G01B 11/26, B65H 63/06**

(21) Numéro de dépôt : **91903791.1**

(22) Date de dépôt : **05.02.91**

(86) Numéro de dépôt international :
**PCT/FR91/00078**

(87) Numéro de publication internationale :
**WO 91/12490 22.08.91 Gazette 91/19**

(54) **PROCEDE ET DISPOSITIF POUR MESURER LA TORSION D'UN FIL TEXTILE.**

(30) Priorité : **06.02.90 FR 9001393**

(43) Date de publication de la demande :
**22.01.92 Bulletin 92/04**

(45) Mention de la délivrance du brevet :
**05.10.94 Bulletin 94/40**

(84) Etats contractants désignés :
**CH DE ES FR GB IT LI**

(56) Documents cités :
**WO-A-89/00215**
**GB-A- 1 484 418**
**GB-A- 1 494 264**

(73) Titulaire : **SUPERBA S.A.**
**13 rue de Pfastatt,**
**B.P. 2156**
**F-68060 Mulhouse Cédex (FR)**

(72) Inventeur : **DURAND, Bernard**
**28, rue du Rhin**
**F-68120 Pfastatt (FR)**

(74) Mandataire : **Nithardt, Roland**
**CABINET NITHARDT & BURKARD S.A.,**
**24 rue de l'Est - B.P. 1445**
**F-68071 Mulhouse Cédex (FR)**

EP 0 466 892 B1

## Description

La présente invention concerne un procédé pour mesurer la torsion d'un fil textile par voie optique, dans lequel on éclaire le fil au moyen d'un faisceau lumineux, pour former une tache lumineuse qui correspond à la lumière diffractée par les fibres de surface de l'âme compacte de ce fil.

Elle concerne également un dispositif pour la mise en oeuvre du procédé pour mesurer la torsion d'un fil textile par voie optique, comprenant des moyens pour éclairer le fil par un faisceau lumineux pour former une tache lumineuse qui correspond à la lumière diffractée par les fibres de surface de l'âme compacte de ce fil.

On connaît plusieurs méthodes pour mesurer la torsion d'un fil textile qui constitue l'une des caractéristiques les plus importantes du fil. En effet, la plupart des propriétés mécaniques et même l'accessibilité des réactifs notamment des colorants pendant la teinture dépendent de ce paramètre fondamental. La torsion se détermine par la formule suivante :

$$T = \mathrm{tg}\alpha\, /\, \pi\, .\, d$$

où T représente la torsion en tours/mètre $\alpha$ l'angle de torsion et d le diamètre du fil en mètre pour autant que la structure soit considérée comme idéale et corresponde à un modèle traditionnel comportant des enroulements hélicoïdaux de fibres en couches superposées. Dans ce cas, l'angle caractéristique de l'enroulement hélicoïdal définit la torsion. L'une des méthodes connue est dite "mesure par détorsion simple".

Cette méthode consiste à détordre le filé jusqu'à la torsion zéro et à relever le nombre de tours nécessaires pour obtenir cette détorsion. Bien que cette mesure soit très simple, en pratique, on rencontre des difficultés dès que l'on s'éloigne des filés peignés classiques et que le filé est très irrégulier, parce qu'il est alors pratiquement impossible de le détordre régulièrement et d'obtenir la torsion zéro.

En effet, dans une structure très irrégulière, tandis que certaines parties sont détordues, d'autres parties sont encore tordues et leur détorsion s'accompagne d'une retorsion en sens inverse des parties déjà détordues.

Pour pallier cet inconvénient, il a été proposé de s'aider d'une aiguille et de la faire glisser entre les fibres sur la distance séparant les deux pinces du torsiomètre. Ceci implique de travailler sur des éprouvettes très courtes de l'ordre de 2,5 cm à 5 cm au maximum. Même dans ce cas, cette détermination est parfois très difficilement réalisable.

Une autre méthode connue est appelée "mesure par détorsion-retorsion". Le principe de la mesure par détorsion-retorsion en sens inverse jusqu'à recouvrement de la longueur initiale, repose sur l'hypothèse de travail selon laquelle le racours provoqué par une torsion est indépendant du sens de la torsion. Ceci ne peut être vrai que dans le cas du filé parfait, filé composé de fibres idéales n'ayant pas de "mémoire" de forme imposée par la torsion.

Dans la pratique, du fait même du stockage, il y a fixation de la position des fibres dans le filé, et par conséquent on a obligatoirement une certaine mémoire de la forme.

Une troisième méthode connue est appelée "mesure par détorsions-retorsions multiples avec correction d'épreuves".

La contre-épreuve a été plusieurs fois suggérée comme moyen pour tendre vers une estimation correcte de la torsion . La détermination par contre-épreuve consiste à faire une détorsion et une retorsion suivies d'une nouvelle détorsion et retorsion dans le sens original jusqu'à recouvrement de la longueur initiale. Cette mesure suppose à nouveau une compensation du comportement par détorsion-retorsion dans les deux sens de la torsion.

La mesure de détorsion-retorsion avec quadruple contre-épreuve est une mesure de détermination de la torsion qui nécessite quatre mesures successives effectuées à l'aide d'un même compteur décompteur. Dans un premier temps, l'éprouvette est détordue puis retordue de façon continue jusqu'à lui faire reprendre sa longueur initiale. On détord le nombre de tours nécessaire pour annuler la torsion, puis on retord du même nombre de tours nécessaire à la retorsion en sens inverse augmenté d'un certain nombre de tours en raison de la symétrie non parfaite des opérations de détorsion-retorsion, notamment de la fixation plus ou moins importante de la torsion sur le fil au moment de la mesure.

Une autre méthode, par voie optique, dite de microscopie, consiste à mesurer l'angle d'inclinaison des spires de fibres par rapport à l'axe du filé et à calculer la torsion T (tours/m) en connaissant le diamètre du filé d (m).

Ceci suppose donc un filé réalisé par couches concentriques de fibres successives et disposées régulièrement ainsi qu'un diamètre du filé rigoureusement défini dans une enveloppe parfaitement cylindrique, ce qui n'est jamais le cas. De plus, la variation relativement importante de la section apparente du fil implique un grand nombre de mesures.

La méthode de PARAMONOV fournit une technique rapide de détermination de l'angle de torsion fondé sur une méthode optique qui utilise la dispersion aux petits angles des rayons laser par les fibres. Pour observer le phénomène, un faisceau laser étroit est orienté perpendiculairement au filé. Les rayons diffractés par le filé sont recueillis par un dispositif placé derrière le fil. Les angles compris entre la ligne centrale et chaque branche correspondant respectivement à la composante due à la torsion des fibres situées sur le bord supérieur du filé et à celle due à la torsion des fibres situées sur le bord inférieur du filé, sont égaux aux angles d'inclinaison des fibres superficielles par rapport à l'axe de part et d'autre du filé.

Cette mesure rapide pour la détermination ponctuelle de la situation des fibres à un niveau donné du filé ne permet d'obtenir qu'une valeur approximative de la torsion superficielle, même si les déterminations sont répétées. Cette mesure ne donne des résultats acceptables qu'avec des filés peignés classiques fabriqués avec grand soin. En réalité, il y a une erreur de principe. En effet, la dispersion est le fruit des fibres marginales de la structure pileuse de l'âme compacte et non des fibres superficielles de l'âme compacte, de sorte que les résultats obtenus sont en général entachés d'erreurs.

Le brevet britannique GB-A-1 484 418 décrit une méthode de détermination d'une interférence due à la distance qui sépare deux irrégularités occasionnées par l'inclinaison des fibres due à la torsion. Cette mesure suppose un filé quasi parfait en diamètre, en organisation et même en régularité.

Le brevet britannique GB-A-1 494 264 décrit un procédé et un dispositif pour déterminer l'angle de torsion d'un fil par éclairement du fil au moyen d'une source de lumière large et analyse de la tache de lumière réfléchie par chaque fibre. L'angle d'inclinaison des fibres ne peut être correctement déterminé que si les fibres sont réfléchissantes et parfaitement organisées, ce qui est le cas uniquement pour les fils à base de fibres ou filaments synthétiques. Dans le cas contraire, pour les fils à base de fibres naturelles, le fil devient un diffuseur de lumière dans lequel aucune orientation priviligiée des fibres ne peut être extraite.

Parmi les méthodes connues, il existe deux catégories d'approche pour la mesure de la torsion :

- sans déformation du filé, on mesure l'angle d'inclinaison à l'aide d'un appareil optique,
- en faisant subir au filé soit une détorsion, soit un ensemble de détorsion (s) - retorsion (s) suivant des conditions données : ce sont les méthodes mécaniques classiques.

Aucune de ces méthodes connue n'est réellement satisfaisante. La présente invention se propose de résoudre le problème de la mesure de la torsion d'une manière satisfaisante et rapide.

Dans ce but, le procédé selon l'invention défini en préambule est caractérisé en ce que l'on examine cette tache lumineuse pour en analyser la répartition énergétique en en ce que l'on définit l'angle de torsion en déterminant la direction du maximum d'énergie dans la tache.

Selon une forme de réalisation préférée on éclaire le fil au moyen d'un faisceau monochromatique de lumière cohérente sensiblement perpendiculaire à la direction principale du fil.

Pour déterminer la direction du maximum d'énergie dans la tache on détermine de préférence la dimension maximale de cette tache, l'angle de torsion étant complémentaire à l'angle que fait la direction dans laquelle la dimension de la tache est maximale avec la direction principale du fil.

Pour rechercher la direction dans laquelle la dimension de la tache est maximale on peut utiliser au moins une fente tournante.

Selon une autre forme de réalisation, on peut rechercher la direction dans laquelle la dimension de la tache est maximale au moyen d'une matrice de détecteurs.

Selon une variante, on peut rechercher la direction dans laquelle la dimension de la tache est maximale au moyen d'une barrette linéaire de détecteurs et d'un faisceau de fibres optiques dont une extrémité comporte une disposition des fibres en couronne et l'autre extrémité une disposition linéaire des fibres.

Selon une autre variante, on peut rechercher la direction dans laquelle la dimension de la tache est maximale au moyen d'un codeur d'angle.

Dans ce but également, le dispositif pour la mise en oeuvre de ce procédé est caractérisé en ce qu'il comporte des moyens pour analyser la répartition énergétique de cette tache lumineuse et pour définir l'angle de torsion par détermination de la direction du maximum d'énergie dans ladite tache.

Selon un mode de réalisation préféré il comporte des moyens pour rechercher la dimension maximale de cette tache.

Les moyens pour rechercher la dimension maximale de la tache lumineuse comprennent un analyseur rotatif comportant au moins une fente tournante.

Selon une variante, les moyens pour rechercher la dimension maximale de la tache lumineuse comprennent une matrice de détecteurs.

Selon une autre forme de réalisation, les moyens pour rechercher la dimension maximale de la tache lumineuse comprennent une barrette linéaire de détecteurs et un faisceau de fibres optiques dont une extrémité comporte une répartition des fibres en couronne et l'autre extrémité une disposition linéaire des fibres.

Selon une autre variante, les moyens pour rechercher la dimension maximale de la tache lumineuse comprennent un codeur d'angle comportant au moins une série de détecteurs discrets disposés sur une ligne circulaire et un circuit électronique pour détecter les détecteurs fournissant le signal maximal.

La présente invention sera mieux comprise en référence à la description d'une forme de réalisation préférée et au dessin annexé dans lequel :

La figure 1 illustre la forme et la disposition d'une tache de diffraction dont l'analyse permet de déduire la torsion du fil, et

la figure 2 illustre un dispositif pour permettre la mesure de la torsion d'un fil textile par le procédé de l'invention.

Dans le cadre de ce procédé on part de l'hypothèse suivante : le fil a une structure relativement simple, à savoir la structure idéalisée du modèle traditionnel qui a des enroulements hélicoïdaux de fibres en couches superposées, de telle sorte que la torsion est définie par l'angle caractéristique de l'enroulement hélicoïdal. La mesure proprement dite est basée sur les théories de la diffraction et de la réflexion. On éclaire le fil au moyen d'un faisceau lumineux qui est par exemple un faisceau de lumière cohérente et monochromatique, ce faisceau étant sensiblement parallèle et étroit. Lorsqu'il atteint les fibres de surface de l'âme compacte du fil, celles-ci diffractent la lumière incidente sur les fibres voisines, ce qui engendre une tache lumineuse sur le fil. L'analyse de la répartition énergétique de cette tache lumineuse permet de déterminer la torsion du fil, la direction principale de cette tache qui a une forme oblongue ou dans certains cas sensiblement elliptique étant celle qui est perpendiculaire aux fibres.

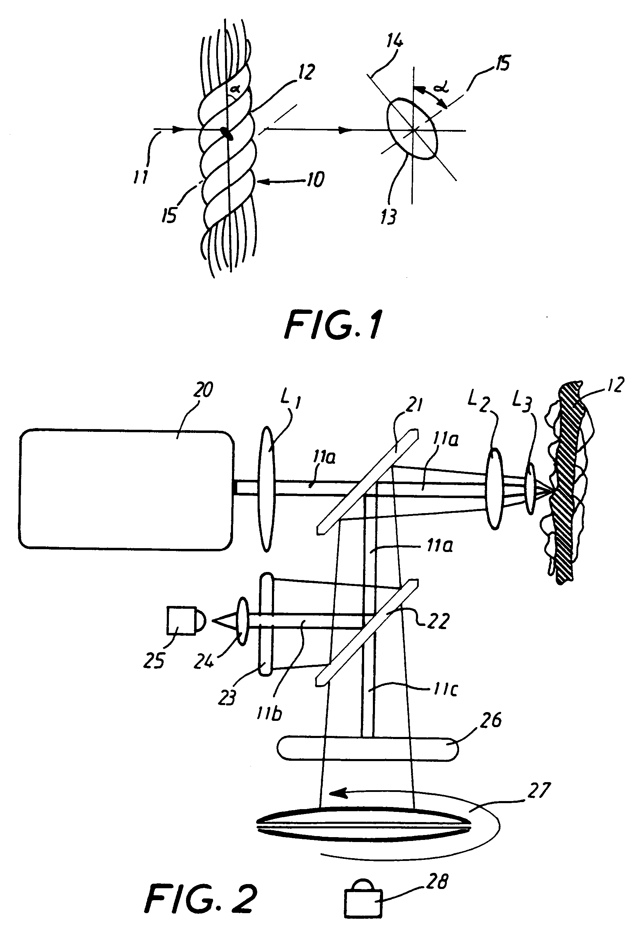

Comme le montre la figure 1, un fil 10 est éclairé par un faisceau de lumière cohérente 11. Les fibres de surface de ce fil diffractent la lumière pour former une tache lumineuse 12 qui peut être reprise et projetée sur un écran pour constituer une image 13 qui présente une forme oblongue, voire elliptique. L'axe principal 14 de la tache 13 est perpendiculaire à la direction 15 des fils. L'angle de torsion est l'angle que fait la direction 15 avec l'axe du fil, c'est-à-dire l'angle complémentaire de celui que fait l'axe principal 14 de la tache lumineuse avec l'axe du fil. La recherche de l'axe principal de la tache, qui se fait par une analyse de la distribution énergétique dans la tache, permet de déterminer l'angle de torsion $\alpha$. La figure 2 illustre une forme de réalisation de l'appareil qui permet d'effectuer la mesure de l'angle de torsion. Il comporte une source laser 20 qui émet le faisceau 11 sur le fil 12, à travers une optique comportant par exemple trois lentilles L1, L2 et L3. Un miroir semi-transparent 21 renvoie le faisceau 11a réfléchi par le fil 12 sur un deuxième miroir semi-transparent 22 qui le divise en un faisceau 11b et un faisceau 11c. Le faisceau 11b est filtré par un filtre 23 et transmis à travers une optique 24 sur un capteur 25 dit capteur de macro-structure. Le faisceau 11c est filtré par un filtre 26 et transmis à un dispositif destiné à rechercher la dimension maximale de la tache lumineuse, qui est par exemple un analyseur rotatif 27 comportant au moins une fente tournante. Un capteur 28 dit capteur de micro-structure capte les signaux transmis par l'analyseur rotatif 27.

Cet analyseur rotatif peut être remplacé par tout autre dispositif de détection approprié, par exemple une matrice de détecteurs, une barrette linéaire de détecteurs combinée avec un faisceau de fibres optiques dont une extrémité présente une répartition des fibres en couronne et l'autre extrémité une disposition linéaire des fibres. Une autre forme de réalisation peut prévoir un codeur d'angle comportant au moins une série de détecteurs discrets disposés sur une ligne circulaire et un circuit électronique pour détecter les détecteurs fournissant le signal maximal.

On notera que le filtre 23 arrête la lumière diffusée par les fibres de surface c'est-à-dire reprend l'image de l'impact du faisceau incident sur l'âme compacte du fil, tandis que le filtre 26 arrête le fond continu, c'est-à-dire la lumière réfléchie directement par l'âme compacte du fil pour reprendre la trace laissée par la diffraction des fibres de l'âme compacte en interaction avec le faisceau incident.

De ce fait, la torsion peut être déterminée pour les fils à fibres torsadées tels que représentés par la figure 1 au moyen du capteur dit de micro-structure. La torsion des fils dits retors qui comportent des brins entrecroisés, peut être déterminée par le capteur dit de macro-structure.

## Revendications

1. Procédé pour mesurer la torsion d'un fil (10) textile par voie optique, dans lequel on éclaire le fil (10) au moyen d'un faisceau lumineux (11), pour former une tache (12) lumineuse qui correspond à la lumière diffractée par les fibres de surface de l'âme compacte de ce fil (10), caractérisé en ce que l'on examine cette tache (12) lumineuse pour en analyser la répartition énergétique et en ce que l'on définit l'angle ($\alpha$) de torsion en déterminant la direction du maximum d'énergie dans la tache (12).

2. Procédé selon la revendication 1, caractérisé en ce que l'on éclaire le fil au moyen d'un faisceau monochromatique de lumière cohérente, sensiblement perpendiculaire à la direction principale du fil.

3. Procédé selon la revendication 1, caractérisé en ce que l'on détermine la direction du maximum d'énergie dans la tache en déterminant la dimension maximale de cette tache, l'angle de torsion étant complémentaire à l'angle que fait la direction dans laquelle la dimension de la tache est maximale avec la direction principale du fil.

4. Procédé selon la revendication 3, caractérisé en ce que l'on recherche la direction dans laquelle la dimension de la tache est maximale au moyen d'au moins une fente tournante.

5. Procédé selon la revendication 3, caractérisé en ce que l'on recherche la direction dans laquelle la dimension de la tache est maximale au moyen d'une matrice de détecteurs.

6. Procédé selon la revendication 5, caractérisé en ce que l'on recherche la direction dans laquelle la dimension de la tache est maximale au moyen d'une barrette linéaire de détecteurs et d'un faisceau de fibres optiques dont une extrémité comporte une disposition des fibres en couronne et l'autre extrémité une disposition linéaire des fibres.

7. Procédé selon la revendication 5, caractérisé en ce que l'on recherche la direction dans laquelle la dimension de la tache est maximale au moyen d'un codeur d'angle.

8. Dispositif pour la mise en oeuvre du procédé selon la revendication 1, pour mesurer la torsion d'un fil textile par voie optique, comprenant des moyens pour éclairer le fil (10) par un faisceau lumineux (11) pour former une tache lumineuse (12) qui correspond à la lumière diffractée par les fibres de surface de l'âme compacte de ce fil, caractérisé en ce qu'il comporte des moyens pour analyser la répartition énergétique de cette tache lumineuse et pour définir l'angle de torsion (α) par détermination de la direction du maximum d'énergie dans ladite tache (12).

9. Dispositif selon la revendication 8, caractérisé en ce qu'il comporte des moyens pour rechercher la dimension maximale de cette tache.

10. Dispositif selon la revendication 9, caractérisé en ce que les moyens pour rechercher la dimension maximale de la tache lumineuse comprennent un analyseur rotatif (27) comportant au moins une fente tournante.

11. Dispositif selon la revendication 9, caractérisé en ce que les moyens pour rechercher la dimension maximale de la tache lumineuse comprennent une matrice de détecteurs.

12. Dispositif selon la revendication 9, caractérisé en ce que les moyens pour rechercher la dimension maximale de la tache lumineuse comprennent une barrette linéaire de détecteurs et un faisceau de fibres optiques dont une extrémité comporte une répartition des fibres en couronne et l'autre extrémité une disposition linéaire des fibres.

13. Dispositif selon la revendication 9, caractérisé en ce que les moyens pour rechercher la dimension maximale de la tache lumineuse comprennent un codeur d'angle comportant au moins une série de détecteurs discrets disposés sur une ligne circulaire et un circuit électronique pour détecter les détecteurs fournissant le signal maximal.

**Patentansprüche**

1. Verfahren zur Messung der Drehung eines Textilfadens (10) auf optischem Wege, indem man den Faden (10) durch ein Lichtbündel (11) beleuchtet, so dass sich ein Lichtfleck (12) bildet, der dem Licht entspricht, das von den Oberflächenfasern des kompakten Kerns des Fadens (10) abgelenkt wird, gekennzeichnet dadurch, dass man diesen Lichtfleck (12) untersucht um dessen Energieaufteilung zu analysieren und man den Drehwinkel (a) definiert, indem man die Richtung der maximalen Energie in dem Lichtfleck (12) feststellt.

2. Verfahren gemäss Anspruch 1, dadurch gekennzeichnet, dass man den Faden mittels eines einfarbigen Bündels kohärenten Lichtes beleuchtet, das wesentlich senkrecht zu der Hauptrichtung des Fadens ist.

3. Verfahren gemäss Anspruch 1, dadurch gekennzeichnet, dass man die Richtung der maximalen Energie in dem Lichtfleck bestimmt, indem man die grösste Abmessung dieses Fleckens feststellt, wobei der Drehungswinkel komplementär zum dem Winkel zwischen der Richtung der grössten Abmessung des Lichtflecks und der Hauptrichtung des Fadens ist.

4. Verfahren gemäss Anspruch 3, gekennzeichnet dadurch, dass man die Richtung feststellt, in der die Abmessung des Lichtflecks am grössten ist, mittels mindestens eines drehbaren Schlitzes.

5. Verfahren gemäss Anspruch 3, gekennzeichnet dadurch, dass man die Richtung feststellt, in der die Abmessung des Lichtflecks am grössten ist, mittels einer Detektormatrize.

6. Verfahren gemäss Anspruch 5, gekennzeichnet dadurch, dass man die Richtung feststellt, in der die Abmessung des Lichtflecks am grössten ist, mittels einer Detektorreihe und eines Bündels Lichtleitfasern, dessen eines Ende eine Anordnung der Fasern in Kronenform und das andere Ende eine lineare Anordnung der Fasern enthält.

7. Verfahren gemäss Anspruch 5, gekennzeichnet dadurch, dass man die Richtung feststellt, in der die Abmessung des Lichtflecks am grössten ist, mittels eines Winkelkodierers.

8. Vorrichtung für die Durchführung des Verfahrens gemäss Anspruch 1 zur Messung der Drehung eines Textilfadens auf optischem Wege, der Mittel zur Beleuchtung des Fadens (10) durch ein Lichtbündel (11) enthält, um einen Lichtflecken (12) zu bilden, der dem Licht entspricht, das von den Oberflächenfasern des kompakten Kerns dieses Fadens abgelenkt wird, gekennzeichnet dadurch dass sie Mittel enthält um die Energieaufteilung dieses Lichtfleckens zu analysieren und den Drehungswinkel (α) durch Bestimmung der Richtung der maximalen Energie in dem besagten Flecken (12) zu definieren.

9. Vorrichtung gemäss Anspruch 8, gekennzeichnet dadurch, dass sie Mittel enthält, um die grösste Abmessung dieses Fadens festzustellen.

10. Vorrichtung gemäss Anspruch 9, gekennzeichnet dadurch, dass die Mittel zur Bestimmung der grössten Abmessung des Lichtfleckens einen drehenden Analysator (27) enthalten, der mindestens einen drehbaren Schlitz enthält.

11. Vorrichtung gemäss Anspruch 9, gekennzeichnet dadurch, dass die Mittel zur Bestimmung der grössten Abmessung des Lichtfleckens eine Detektormatrize enthalten.

12. Vorrichtung gemäss Anspruch 9, gekennzeichnet dadurch, dass die Mittel zur Bestimmung der grössten Abmessung des Lichtfleckens eine Detektorreihe und ein Bündel Lichtleitfasern enthalten, dessen eines Ende eine Aufteilung der Fasern in Kronenform und das andere Ende eine lineare Anordnung der Fasern enthält.

13. Vorrichtung gemäss Anspruch 9, gekennzeichnet dadurch, dass die Mittel zur Bestimmung der grössten Abmessung des Lichtfleckens einen Winkelkodierer enthalten, mit mindestens einer Serie getrennter Detektoren, die auf einer runden Linie angeordnet sind, sowie einen elektronischen Kreislauf zur Erfassung der Detektoren, die das stärkste Signal liefern.

**Claims**

1. Process to measure the twist of a textile thread (10) by means of an optical path in which one illuminates the thread (10) using a light beam (11), to form a light spot (12) which corresponds to the light diffracted by the surface fibres of the compact core of this thread (10), characterised in that one examines this light spot (12) in order to analyse the energetic distribution and in that one defines the twist angle (α) by determining the direction of maximum energy of the spot (12).

2. Process according to claim 1, characterised in that the thread is illuminated by a mono-chrome beam of coherent light, considerably perpendicular to the main direction of thread.

3. Process according to claim 1, characterised in that one determines the direction of maximum energy in the spot by determining the maximum size of this spot, the twist angle being complimentary to the angle formed between the direction of maximum size of spot and the main direction of thread.

4. Process according to claim 3, characterised in that one establishes the direction in which the spot is at its largest by means of a rotating slot.

5. Process according to claim 3, characterised in that one establishes the direction in which the spot is at its largest by means of a sensor matrix.

6. Process according to claim 5, characterised in that one establishes the direction in which the spot is at its largest by means of a row of sensors and a bundle of optic fibres forming at one end a crown of fibres and at the other end a row of fibres.

7. Process according to claim 5, characterised in that one establishes the direction in which the spot is at its largest by means of an angular encoder.

8. Device for application of process according to claim 1, to measure the twist of a textile thread by means of an optical path comprising a means of illuminating the thread (10) using a light beam (11), to form a light spot (12) which corresponds to the light diffracted by the surface fibres of the compact core of this thread, characterised in that it comprises a means to analyse the energetic distribution in this spot and to define the twist angle (α) by determining the direction of maximum energy of the said spot (12).

9. Device according to claim 8, characterised in that it contains a means to establish the maximum size of spot.

10. Device according to claim 9, characterised in that the means to establish the maximum size of the light spot comprises a rotary analyser (27) foreseen with at least one rotating slot.

11. Device according to claim 9, characterised in that the means to establish the maximum size of the light spot comprises a sensor matrix.

12. Device according to claim 9, characterised in that the means to establish the maximum size of the light spot comprises a row of sensors and a bundle of optic fibres forming at one end a crown of fibres and at the other end a row of fibres.

13. Device according to claim 9, characterised in that the means to establish the maximum size of the light spot comprises an angular encoder with at least one series of discrete sensors placed along a circular line and an electronic circuit to detect the sensors supplying the maximum signal.

FIG.1

FIG. 2